# EUROPEAN PATENT APPLICATION

(11) **EP 4 736 906 A1**
(43) Date of publication of application: **06.05.2026**
(21) Application number: 25208845.5
(22) Date of filing: 15.10.2025
(51) Int. Cl.: A61M 1/36

(54) **EXTRACORPOREAL LIFE SUPPORT SYSTEM WITH AIR PURGE CONTROL**

(30) Priority: 31.10.2024 US 202418933655
(71) Applicant: LivaNova Deutschland GmbH, 80939 Munich (DE)
(72) Inventor: Schubert, Friedemann, 80639 München (DE); Kohnen, Lena, 81369 München (DE); Stoll, Sascha Ewald, 82205 Gilching (DE)
(74) Representative: Grünecker Patent- und Rechtsanwälte PartG mbB

(57) **Abstract**

Extracorporeal blood treatment systems including air purge control capabilities are disclosed. An example extracorporeal blood treatment system may include an extracorporeal blood pathway between a venous side of the heart and an arterial side of the heart, an air trap positioned along the blood pathway, a first sensor positioned adjacent the bubble trap and a second sensor positioned adjacent the bubble trap. Further, a first signal sent by the first sensor is configured to activate air removal from the air trap and a second signal sent by the second sensor is configured to deactivate air removal from the air trap.

## Description

### TECHNICAL FIELD

The present disclosure relates to an extracorporeal life support system and methods for manufacturing and/or using an extracorporeal life support system. More particularly, the present disclosure relates to an extracorporeal life support system including air purge control capabilities and methods for manufacturing and/or using an extracorporeal life support system.

### BACKGROUND

Some medical procedures (e.g., medical procedures which treat cardiac or respiratory disease) may require the use of a life support system that supports cardiac and pulmonary functions by artificially supporting the heart and the lung function. In some instances, this may be carried out by an extracorporeal perfusion system. An extracorporeal perfusion system may provide both cardiac and respiratory support to a patient whose heart and lungs are unable to provide an adequate amount of gas exchange during a cardiac and pulmonary procedure. Extracorporeal perfusion works by removing blood from a patient's body to oxygenate the red blood cells while also removing carbon dioxide. The oxygenated blood is then returned to the patient.

Extracorporeal perfusion systems may include multiple devices that together form a blood recirculation loop between the patient and a blood oxygenator. For example, some extracorporeal perfusion systems may include a blood pump to power blood flow, an oxygenator to oxygenate the blood, a device to filter the blood (which may be included within the oxygenator in some systems), one or more sensors positioned at various locations along blood pathways, one or more clamps and one or more control units. It can be appreciated that a blood pathway (e.g., tubing) may extend from the patient towards a blood pump, then pass through the oxygenator and close the loop by returning to the patient. Accordingly, the blood pump may assist the heart by pumping blood through the circulation loop, while the oxygenator may assist the lungs by oxygenating blood that is eventually returned to the patient.

It can be further appreciated that removing the blood from the patient may include placing multiple cannulas into the patient's body in a variety of locations, depending on the type of medical procedure being performed. For example, to infuse oxygen-rich blood into the patient's body, an arterial cannula may be placed in the ascending aorta of the patient. Additionally, to remove oxygen-rich blood from the patient's body a venous cannula may be placed in the superior vena cava and/or inferior vena cava of the patient.

Further, the extracorporeal perfusion system may be configured to prevent hemolysis by minimizing contact of the blood to air during the placement of the venous cannula. However, in some instances, air may inadvertently enter the venous blood pathway. For example, air may enter the venous blood pathway during placement of the venous cannula. Additionally, air may enter the venous blood pathway via holes within one or more tubes of the tubing system defining the venous blood pathway. Accordingly, it can be appreciated that the extracorporeal perfusion system may include one or more systems configured to remove air which has entered the venous blood pathway. For example, the extracorporeal perfusion system may include an air trap (e.g., air bubble trap) positioned along the venous blood pathway, whereby the air trap is configured to remove air which has entered the venous blood pathway. Extracorporeal perfusion systems including air purge control systems configured to remove air from the venous blood pathway are disclosed herein.

### SUMMARY

An example extracorporeal blood treatment system may include an extracorporeal blood pathway between a venous side of the heart and an arterial side of the heart, an air trap positioned along the blood pathway, a first sensor positioned adjacent the bubble trap and a second sensor positioned adjacent the bubble trap. Further, a first signal sent by the first sensor is configured to activate air removal from the air trap and a second signal sent by the second sensor is configured to deactivate air removal from the air trap.

In addition or alternatively to any example described herein, wherein the first sensor is configured to sense a presence of air in blood flowing along the blood pathway.

In addition or alternatively to any example described herein, wherein the second sensor is configured to sense a change in a gas volume fraction of blood passing along a tubing pathway extending between the air trap and an air removal element.

In addition or alternatively to any example described herein wherein the air removal element includes a vacuum device.

In addition or alternatively to any example described herein, wherein the air removal element includes a roller pump.

In addition or alternatively to any example described herein, wherein the first sensor is positioned upstream of the air trap.

In addition or alternatively to any example described herein, further comprising a tubing pathway having a first end attached to the air trap and a second end attached to an air removal element, and wherein the second sensor is positioned along the tubing pathway.

In addition or alternatively to any example described herein, wherein the second sensor is directly attached to the air trap.

In addition or alternatively to any example described herein, further comprising an air removal element coupled to the air trap, and wherein the activation of the air removal element is configured to generate a vacuum force within the air trap.

In addition or alternatively to any example described herein, further comprising a control unit in communication with the first sensor, the second sensor and the air removal element.

In addition or alternatively to any example described herein, wherein the first sensor is configured to transmit a first signal to the control unit, wherein the second sensor is configured to transmit a second signal to the control unit, wherein the air removal element is configured to receive a third signal corresponding to the first signal from the control unit, and wherein the air removal element is configured to receive a fourth signal corresponding to the second signal from the control unit, and wherein the air control element is configured to adjust a vacuum force in the air trap after receiving the third signal, the fourth signal or both the third and the fourth signals.

In addition or alternatively to any example described herein, wherein the second sensor includes a first level sensor.

In addition or alternatively to any example described herein, wherein the first level sensor is directly attached to the air trap.

In addition or alternatively to any example described herein, wherein the first level sensor is configured to sense a minimum fluid level of blood in the air trap.

In addition or alternatively to any example described herein, wherein the first level sensor is configured to send a signal corresponding to the minimum fluid level of blood in the air trap.

In addition or alternatively to any example described herein, further comprising a third sensor, and wherein the third sensor includes a second level sensor.

In addition or alternatively to any example described herein, wherein the second level sensor is configured to sense a maximum fluid level of blood in the air trap.

In addition or alternatively to any example described herein, wherein the second level sensor is configured to send a signal corresponding to the maximum fluid level of blood in the air trap.

Another example extracorporeal blood treatment system includes an extracorporeal blood pathway between a venous side of the heart and an arterial side of the heart, an air trap positioned along the blood pathway, a first sensor positioned adjacent the air trap, a second sensor positioned adjacent the air trap, a roller pump coupled to the air trap and a control unit in communication with the first sensor, the second sensor and the roller pump. Further, the roller pump is configured to activate after receiving a first signal from the first sensor and to at least partially deactivate after receiving a second signal from the second sensor.

Another example extracorporeal blood treatment system includes an extracorporeal blood pathway between a venous side of the heart and an arterial side of the heart, an air trap positioned along the blood pathway, a first sensor positioned upstream of the air trap, a tubing pathway extending between the air trap and a roller pump, a second sensor positioned within the tubing pathway and a control unit in communication with the first sensor, the second sensor and the roller pump. Further, the first sensor is configured to send a first signal to the control unit, wherein the first signal corresponds to an amount of air present in blood flowing within the blood pathway. Further, the second sensor is configured to send a second signal to the control unit, wherein the second signal corresponds to an amount of air present in the tubing pathway. Further, the roller pump is configured to activate after receiving a third signal from the control unit, wherein the third signal corresponds to the first signal received from the first sensor. Further, the roller pump is configured to at least partially deactivate after receiving a fourth signal from the control unit, and wherein the fourth signal corresponds to the second signal received from the second sensor.

The above summary of some embodiments, aspects, and/or examples is not intended to describe each disclosed embodiment or every implementation of the present disclosure. The figures and detailed description which follow more particularly exemplify these embodiments.

### BRIEF DESCRIPTION OF THE DRAWINGS

The disclosure may be more completely understood in consideration of the following detailed description in connection with the accompanying drawings, in which:
FIG. 1 illustrates an example extracorporeal blood treatment system;
FIG. 2 is a schematic diagram of a computing device;
FIG. 3 illustrates an example extracorporeal blood treatment system;
FIG. 4 illustrates an example air trap;
FIG. 5 illustrates an example extracorporeal blood treatment system;
FIG. 6 illustrates an example air trap.

While aspects of the disclosure are amenable to various modifications and alternative forms, specifics thereof have been shown by way of example in the drawings and will be described in detail. It should be understood, however, that the intention is not to limit aspects of the disclosure to the particular embodiments described. On the contrary, the intention is to cover all modifications, equivalents, and alternatives falling within the spirit and scope of the disclosure.

### DETAILED DESCRIPTION

For the following defined terms, these definitions shall be applied, unless a different definition is given in the claims or elsewhere in this specification.

All numeric values are herein assumed to be modified by the term "about", whether or not explicitly indicated. The term "about" generally refers to a range of numbers that one of skill in the art would consider equivalent to the recited value (e.g., having the same function or result). In many instances, the terms "about" may include numbers that are rounded to the nearest significant figure.

The recitation of numerical ranges by endpoints includes all numbers within that range (e.g. 1 to 5 includes 1, 1.5, 2, 2.75, 3, 3.80, 4, and 5). As used in this specification and the appended claims, the singular forms "a", "an", and "the" include plural referents unless the content clearly dictates otherwise. As used in this specification and the appended claims, the term "or" is generally employed in its sense including "and/or" unless the content clearly dictates otherwise.

It is noted that references in the specification to "an embodiment", "some embodiments", "other embodiments", etc., indicate that the embodiment described may include one or more particular features, structures, and/or characteristics. However, such recitations do not necessarily mean that all embodiments include the particular features, structures, and/or characteristics. Additionally, when particular features, structures, and/or characteristics are described in connection with one embodiment, it should be understood that such features, structures, and/or characteristics may also be used in connection with other embodiments whether or not explicitly described unless clearly stated to the contrary.

The following detailed description should be read with reference to the drawings in which similar elements in different drawings are numbered the same. The drawings, which are not necessarily to scale, depict illustrative embodiments and are not intended to limit the scope of the disclosure.

In a normal heart, blood circulates via a closed path whereby deoxygenated (venous) blood enters the right atrium via both the superior vena cava and inferior vena cava. The venous blood then passes through the right ventricle and is pumped via the pulmonary artery to the lungs, where it absorbs oxygen and releases carbon dioxide. After absorbing oxygen and releasing carbon dioxide in the lungs, the blood becomes oxygenated arterial blood. The oxygenated blood is then returned via the pulmonary veins to the left atrium and is passed to the left ventricle. The oxygenated arterial blood is then pumped through the aorta and eventually throughout the body.

It can be appreciated that if the lungs are incapable of sufficiently oxygenating blood and/or removing carbon dioxide, an oxygenator located outside the body may be used to oxygenate the blood and/or remove carbon dioxide. As discussed above, extracorporeal perfusion is a breathing and heart pumping life support system that may be utilized to support patients while medical treatments (e.g., heart surgery) are performed to treat their underlying illness. When supported via an extracorporeal perfusion system, oxygenation of the patient's blood and removal of carbon dioxide may occur outside the body.

Extracorporeal perfusion is generally performed using a heart-lung bypass system, which may be referred to as a "circuit." The circuit may include a blood flow path exterior of the patient, such as one or more tubing pathways designed to transfer blood from a patient's body to the oxygenator and back into the patient. As described above, the oxygenator may add oxygen to the blood while also removing carbon dioxide (e.g., the oxygenator performs the function of a healthy lung).

In some examples, an extracorporeal perfusion circuit may include a blood pump, oxygenator, tubing pathways (for transfer to and from the body), sensors (e.g., flow, pressure, bubble, temperature, oxygen, carbon dioxide, etc.), remote control clamps, a control unit, and arterial and/or venous access points for the collection of blood in the circuit. It can be appreciated that the function of the blood pump may be to generate blood flow within the extracorporeal perfusion circuit (e.g., circulate blood from the patient to the oxygenator and back to the patient) and to also generate blood pressure within the patient's vascular system. The blood pump may be positioned in the tubing pathway between the patient and the oxygenator. In some extracorporeal perfusion systems a roller pump may be utilized to generate blood flow within the extracorporeal perfusion circuit. However, in other extracorporeal perfusion systems, other blood pumps, including centrifugal pumps may be utilized to generate blood flow within the extracorporeal perfusion circuit.

In some extracorporeal perfusion systems, the oxygenator may include a housing having multiple chambers or pathways separated by a semi-permeable membrane, whereby the patient's blood may flow through one chamber or pathway, while an oxygen gas mixture (i.e., sweep gas) flows through another chamber or pathway. The semi-permeable membrane may include multiple microporous hollow fibers, each fiber having a lumen extending therethrough through which the oxygen gas mixture flows. The gas exchange may occur via diffusion of the gases across multiple microporous fibers, whereby oxygen moves from the inside of the hollow fibers into the blood while carbon dioxide diffuses from the blood into the interior of the hollow fibers, where it is swept away by the sweep gas flowing through the fiber. This gas exchange allows for oxygenation of venous blood and removal of carbon dioxide. In some extracorporeal perfusion systems, the oxygenator may include integrated heat exchangers that allow circulating blood to be cooled and/or warmed prior to returning to the patient.

In some instances, it may be desirable to control one or more parameters of blood flow through the various blood pathways of the extracorporeal perfusion system. For example, it may be desirable to control the flowrate of blood within one or more blood pathways within the extracorporeal perfusion system. In some examples, the flowrate of blood within the blood pathways of the extracorporeal perfusion system may be controlled via a combination of clamps, sensors and pumps, one or more of which may be coupled to the various blood pathways of the extracorporeal perfusion system. In some examples, one or more of the clamps, sensors and pumps may be in communication (e.g., wireless, wired communication, or other communication means capable of transmitting signals) with a control unit, whereby the control unit may be configured to operate one or more of the clamps, sensors and pumps in various combinations to control the flow of blood within the blood pathways of the extracorporeal perfusion system.

Additionally, in some instances air, such as an air bubble, may enter the arterial blood pathway and/or the venous blood pathway during placement of the arterial cannula and/or venous cannula, via the oxygenator or via holes within one or more tubes of the tubing system defining the arterial blood pathway and/or the venous blood pathway. Accordingly, it can be appreciated that the extracorporeal perfusion system may include one or more systems configured to remove air and/or an air bubble which has entered the venous blood pathway. For example, the extracorporeal perfusion system may include an air trap (e.g., air bubble trap) positioned along the venous blood pathway, whereby the air trap is configured to remove air and/or an air bubble which has entered the venous blood pathway. For example, an air trap may be positioned along the venous blood pathway, whereby the air trap may be evacuated via a variety of vacuum systems, including a roller pump or a syringe.

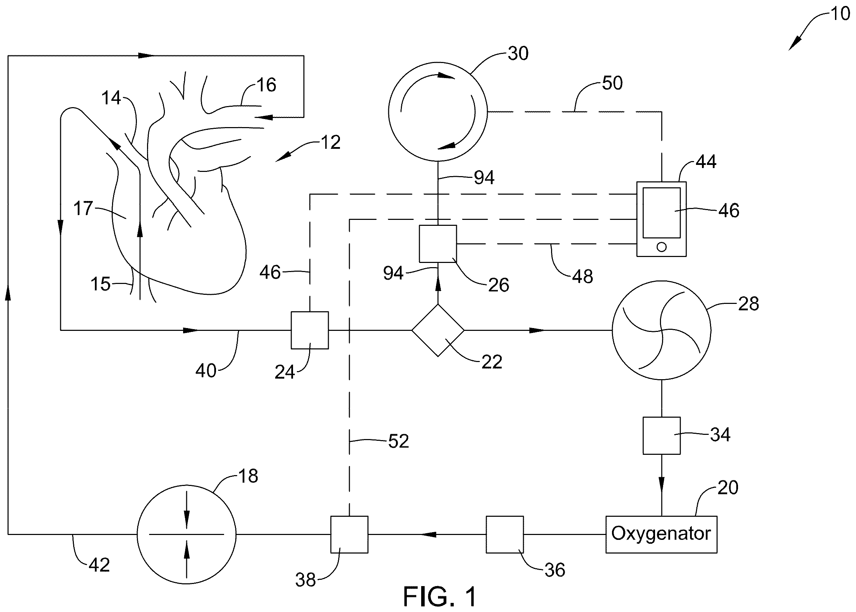

FIG. 1 illustrates an extracorporeal perfusion system 10. The extracorporeal perfusion system 10 may include an oxygenator 20, an arterial clamp 18, an arterial flow sensor 36, an arterial pressure sensor 34, a pump 28 (e.g., arterial pump), a first venous air bubble sensor 24, a second venous air bubble sensor 26, an arterial air bubble sensor 38, an air trap 22 (e.g., air bubble trap), a roller pump 30 and a control unit 44. Additionally, the extracorporeal perfusion system 10 may include one or more blood pathways extending between various components of the extracorporeal perfusion system 10.

The extracorporeal perfusion system 10 may be designed to hold blood which is removed (e.g., via gravity or suction) from a patient via a patient's superior vena cava (SVC) 14 and inferior vena cava (IVC) 15 or, alternatively, from a single cannula placed in the patient's right atrium 17. Generally, blood may then travel to an arterial pump 28 along a venous blood pathway 40. The venous blood pathway 40 may be defined as the blood pathway 40 along which non-oxygenated blood exiting the patient flows toward the oxygenator 20. The arterial pump 28 may then pump the blood toward and into an oxygenator 20. The arterial pump 28 may be configured to deliver blood to the arterial side of the patient. Further, after gas exchange takes place within the semi-permeable membrane of the oxygenator 20, the post-oxygenated blood may travel toward the patient along an arterial blood pathway 42 and return to the patient via a cannula placed in the aorta 16. The arterial blood pathway 42 may be defined as the blood pathway 42 along which oxygenated blood exiting the oxygenator 20 flows back to the aorta 16 of the patient.

As discussed herein, the extracorporeal perfusion system 10 shown in FIG. 1 may also include an arterial clamp 18, arterial flow sensor 36, arterial pressure sensor 34 positioned along the arterial blood pathway 42, whereby the sensors 34, 36 control the arterial pump 28 and help regulate the flow of blood through the arterial blood pathway 42. It can be appreciated that the arterial bubble sensor 38 may be configured to close the arterial clamp 18 if it detects air in the arterial blood pathway 42. Further, the arterial clamp 18 may be configured to close if a user stops the arterial pump 28.

When positioned along the arterial blood pathway 42, the clamp 18 may be configured to actuate such that the clamp 18 decreases or increases the cross-sectional area of a component defining the arterial blood pathway 42. For example, the arterial blood pathway 42 may be formed from a tubing having a wall and a lumen extending therein. The lumen of the tubing may have a cross-sectional area which, along with the velocity of the blood flowing through the tubing, defines the volume of blood which may pass through the tubing over a given time period. It can be appreciated that actuation of the clamp 18 may either clamp down and restrict the cross-sectional area of the tubing or may release and expand the cross-sectional area of the tubing defining the blood pathway 42. In other words, the clamp 18 may be designed to physically deform the tubing to adjust the cross-sectional area of the lumen (which may, in turn, increase the resistance of the tubing), and therefore, the flowrate of blood through the tubing. Further, in some examples, the arterial flow sensor 36 may be positioned on an inner surface, the outer surface or within a wall of the tubing defining the arterial blood pathway 42. In other examples, the arterial flow sensor 36 may be positioned adjacent to a component (e.g., tubing) defining the arterial blood pathway 42.

The arterial flow sensor 36 may be a flow sensor configured to sense (e.g., detect, measure, compute, monitor, etc.) the flowrate of blood passing through the blood pathway 42. In some examples, the arterial flow sensor 36 may be positioned downstream of the oxygenator 20. The arterial flow sensor 36 may be configured to sense the flow rate of blood after it has passed through the oxygenator 20. In some examples, the arterial flow sensor 36 may be configured to communicate with the arterial pump 28, whereby the speed of the arterial pump 28 may be adjusted based on the flow rate of the blood sensed by the arterial flow sensor 36.

Additionally, the extracorporeal perfusion system 10 may include additional sensors positioned along the arterial blood pathway 42. For example, the extracorporeal perfusion system 10 may include one or more sensors for monitoring pressures, temperatures, air in the form of air bubbles, oxygen, oxygen saturation and/or concentration, carbon dioxide, blood gases, or other blood parameters. Further, in some instances a single sensor may be configured to sense multiple blood parameters including blood flowrate, pressures, temperatures, air in the form of bubbles, oxygen saturation and/or concentration, carbon dioxide, blood gases, etc.

FIG. 1 further illustrates that the extracorporeal perfusion system 10 may include an arterial pressure sensor 34 positioned along the arterial blood pathway 42. In some examples, the arterial pressure sensor 34 may be positioned on an inner surface, the outer surface or within a wall of the tubing defining the arterial blood pathway 42. In other examples, the arterial pressure sensor 34 may be positioned adjacent to a component (e.g., tubing) defining the blood pathway 42. As illustrated in FIG. 1, in some examples, the arterial pressure sensor 34 may be positioned between the arterial pump 28 and the oxygenator 20. The arterial pressure sensor 34 may be configured to sense (e.g., detect, measure, compute, monitor, etc.) the pressure of blood passing through the arterial blood pathway 42 between the pump 28 and the oxygenator 20. The arterial pressure sensor may be configured to communicate and control the performance of the arterial pump 28 based on the pressure of blood passing through the arterial blood pathway 42 between the pump 28 and the oxygenator 20.

FIG. 1 further illustrates that the extracorporeal perfusion system 10 may include an air trap 22 (e.g., air bubble trap) positioned along the venous blood pathway 40. In some examples, the air trap 22 (e.g., air bubble trap) may be positioned along the venous blood pathway 40 upstream of the arterial pump 28. It can be appreciated that the air trap 22 (e.g., air bubble trap) may be configured to remove unwanted air (e.g., air bubbles) which may have entered the tubing system of the extracorporeal perfusion system 10. As discussed herein, air may enter the arterial blood pathway 42 and/or the venous blood pathway 40 during placement of the arterial cannula and/or venous cannula, respectively. Further, air may also enter the arterial blood pathway 42 via the oxygenator. Further yet, air may enter the arterial blood pathway 42 and/or the venous blood pathway 40 via holes within one or more tubes of the tubing system defining the arterial blood pathway 42 and/or the venous blood pathway 40.

It can be further appreciated that the air trap 22 (e.g., air bubble trap) may be evacuated via a vacuum or suction device. For example, FIG. 1 illustrates that the extracorporeal perfusion system 10 may further include a roller pump 30 which is coupled (e.g., via tubing) to the air trap 22 (e.g., air bubble trap). The roller pump 30 may be configured to generate a vacuum within the air trap 22 (e.g., air bubble trap), whereby unwanted air which passes into the air trap 22 (e.g., air bubble trap) is pulled out of the air trap 22 (e.g., air bubble trap). As will be discussed in greater detail below, it can be appreciated that utilizing the roller pump 30 to pull air out of the air trap 22 (e.g., air bubble trap) may remove unwanted air which may have inadvertently entered one or more portions of the tubing system of the extracorporeal perfusion system 10.

In some examples, it can be appreciated that a syringe may be utilized in lieu of the roller pump 30 described herein. For example, it can be appreciated that a syringe may be coupled (e.g., via tubing) to the air trap 22 (e.g., air bubble trap), whereby the syringe may be configured to generate a vacuum within the air trap 22 (e.g., air bubble trap). It can be appreciated that air that passes into the air trap 22 (e.g., air bubble trap) may be pulled out of the air trap 22 (e.g., air bubble trap) by the syringe. Like that discussed above, it can be appreciated that utilizing a syringe to pull air out of the air trap 22 (e.g., air bubble trap) may remove unwanted air which may have inadvertently entered one or more portions of the tubing system of the extracorporeal perfusion system 10.

FIG. 1 further illustrates that the extracorporeal perfusion system 10 may further include a first bubble sensor 24 positioned along the venous blood pathway 40. In some examples, the first bubble sensor 24 may be positioned between the patient and the air trap 22 (e.g., air bubble trap).

Additionally, FIG. 1 further illustrates that the extracorporeal perfusion system 10 may further include a second bubble sensor 26 positioned along the venous blood pathway 40. In some examples, the second bubble sensor 26 may be positioned between the air trap 22 (e.g., air bubble trap) and the roller pump 30 (or syringe if used in lieu of the roller pump 30) within a tubing pathway that extends between the air trap 22 (e.g., air bubble trap) and the roller pump 30 (or syringe if used in lieu of the roller pump 30). In other examples, the second bubble sensor 26 may be directly attached to the air trap 22 (e.g., air bubble trap).

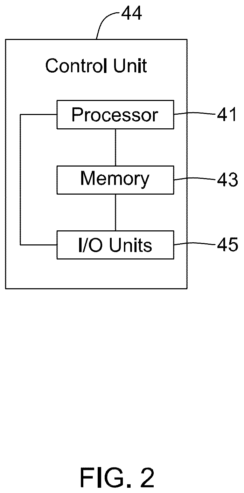

As discussed herein, the extracorporeal perfusion system 10 may also include a control unit 44. The control unit 44 may include a visual display 46 and/or one or more control knob (e.g., buttons). FIG. 2 further illustrates that the control unit 44 may include, among other suitable components, a processor 41, memory 43, and an I/O unit 45.

The processor 41 of the control unit 44 may include a single processor or more than one processor working individually or with one another. The processor 41 may be configured to execute instructions, including instructions that may be loaded into the memory 43 and/or other suitable memory. Example processor components may include, but are not limited to, microprocessors, microcontrollers, multi-core processors, graphical processing units, digital signal processors, application specific integrated circuits (ASICs), field programmable gate arrays (FPGAs), discrete circuitry, and/or other suitable types of data processing devices.

The memory 43 of the control unit 44 may include a single memory component or more than one memory component each working individually or with one another. Example types of memory may include random access memory (RAM), EEPROM, FLASH, suitable volatile storage devices, suitable non-volatile storage devices, persistent memory (e.g., read only memory (ROM), hard drive, Flash memory, optical disc memory, and/or other suitable persistent memory) and/or other suitable types of memory. The memory 43 may be or may include a non-transitory computer readable medium.

The I/O units 45 of the control unit 44 may include a single I/O component or more than one I/O component each working individually or with one another. Example I/O units 45 may be any type of communication port configured to communicate with other components of the building management system. Example types of I/O units 45 may include wired ports, wireless ports, radio frequency (RF) ports, Low-Energy Bluetooth ports, Bluetooth ports, Near-Field Communication (NFC) ports, HDMI ports, Wi-Fi ports, Ethernet ports, VGA ports, serial ports, parallel ports, component video ports, S-video ports, composite audio/video ports, DVI ports, USB ports, optical ports, and/or other suitable ports.

Additionally, FIG. 1 illustrates that the control unit 44 may be in communication with various components of the extracorporeal perfusion system 10. For example, FIG. 1 illustrates that the control unit 44 may be in communication with the first bubble sensor 24, the second bubble sensor 26, the arterial bubble sensor 38 and the roller pump 30. FIG. 1 depicts the first bubble sensor 24 in communication with the control unit 44 via a dashed line 46. Further, FIG. 1 depicts the second bubble sensor 26 in communication with the control unit 44 via a dashed line 48. Further yet, FIG. 1 depicts the arterial bubble sensor 38 in communication with the control unit 44 via a dashed line 52. Further yet, FIG. 1 depicts the roller pump 30 in communication with the control unit 44 via a dashed line 50. In some examples, the control unit 44 may be integrated into a console or workstation of the extracorporeal perfusion system 10. In other examples, the control unit 44 may be integrated directly into a heart-lung machine. The control unit 44 may be in direct or indirect communication with a console, workstation and/or a heart-lung machine.

Additionally, the control unit 44 may be in communication with other components of the extracorporeal perfusion system 10. For example, the control unit 44 may be in communication (e.g., wireless, wired communication, or other communication means capable of transmitting signals) with the arterial clamp 18, the arterial flow sensor 36 and the arterial pressure sensor 34 positioned along the arterial blood pathway 42.

It can be appreciated that together the components of the extracorporeal perfusion system 10 described herein may form a closed-loop system capable of automatically or manually regulating the flowrate of blood within the venous blood pathway 40 and the arterial blood pathway 42. As discussed herein, it can be appreciated that regulating the flowrate of blood within the extracorporeal perfusion system 10 may include removing unwanted air from the venous blood pathway 40 and/or the arterial blood pathway 42.

As discussed herein, FIG. 1 illustrates that the extracorporeal perfusion system 10 described herein may include a first venous bubble sensor 24 positioned upstream of the air trap 22 (e.g., air bubble trap). Further, FIG. 1 illustrates that the air trap 22 (e.g., air bubble trap) may be positioned upstream of the arterial pump 28. It can be appreciated that when the first venous bubble sensor 24 detects the presence of air in the venous blood pathway 40, it may communicate (via the control unit 44) to start the roller pump 30. It can be appreciated that starting the roller pump 30 may begin to remove (e.g., purge) air from the venous blood pathway 40.

In some examples, the roller pump 30 may be pre-programmed to run at a preset speed for a preset time as soon at first venous bubble sensor 24 detects air in the venous blood pathway 40 and triggers the roller pump 30 to start running. In some examples, a user may manually program the time for which the roller pump 30 may run after being triggered by the first venous bubble sensor 24. Further, in some examples, if the first venous bubble sensor 24 continues to detect air in the venous blood pathway 40, the programmed timer which controls roller pump 30 may be reset to the beginning and will continue to run its full pre-programmed timing cycle until completion. In some instances, this may result in the roller pump 30 running beyond what is necessary to remove the unwanted air from the venous blood pathway 40.

Accordingly, FIG. 1 illustrates that, in some examples, the extracorporeal perfusion system 10 may include a second venous bubble sensor 26 positioned between the air trap 22 (e.g., air bubble trap) and the roller pump 30. Any of the sensors described herein (including the first venous bubble sensor 24 and the second venous bubble sensor 26) may be configured to detect a change in the volume fraction of air (e.g., oxygen, carbon dioxide, etc.) and/or air in the form of air bubbles and/or dissolved gases (e.g., oxygen, carbon dioxide, etc.) in blood. The volume fraction of air in the blood may be defined herein as the proportion of the total volume of the blood that is occupied by air, air in the form of air bubbles and/or dissolved gases. In some examples, any of the sensors described herein (including the first venous bubble sensor 24 and the second venous bubble sensor 26) may include an ultrasonic sensor configured to detect a change in the volume fraction of air (e.g., oxygen, carbon dioxide, etc.), air in the form of air bubbles and/or dissolved gases in blood. Further, any of the sensors described herein (including the first venous bubble sensor 24 and the second venous bubble sensor 26) may include a microprocessor configured to detect a change in the volume fraction of air (e.g., oxygen, carbon dioxide, etc.), air in the form of air bubbles and/or dissolved gases in blood and send out a signal corresponding to the change. In some examples, any of the sensors described herein (including the first venous bubble sensor 24 and the second venous bubble sensor 26) may be configured to detect the presence and/or absence of air bubbles in blood. Further yet, in some examples, any of the sensors described herein (including the first venous bubble sensor 24 and the second venous bubble sensor 26) may include an ultrasonic sensor configured to detect a change in the density of air (e.g., oxygen, carbon dioxide, etc.), air in the form of air bubbles and/or dissolved gases in blood.

In this configuration, the roller pump 30 may be started when the first venous bubble sensor 24 detects the presence of air in the venous blood pathway 40. However, as the roller pump 30 begins pulling air out of the air trap 22 (e.g., air bubble trap) and into the tubing 94 leading to the roller pump 30, the second venous bubble sensor 26 may begin sensing a change in the volume fraction of air (e.g., oxygen, carbon dioxide, etc.) and/or air in the form of air bubbles in the tubing 94 leading to the roller pump 30. Accordingly, when the second venous bubble sensor 26 senses the change in the volume fraction of air (e.g., oxygen, carbon dioxide, etc.) and/or air in the form of air bubbles in the tubing 94 leading to the roller pump 30, it may send a signal to the roller pump 30 (via the control unit 44) to stop pumping, thereby stopping air from being purged from the air trap 22 (e.g., air bubble trap). It can be appreciated that with sensing and communication from both the first venous bubble sensor 24 and the second venous bubble sensor 26, the roller pump 30 may run for the minimum amount of time to remove the undesired air from the venous blood pathway 40. This is in contrast to a system with only one bubble sensor, in which the roller pump 30 may run until the pre-programmed timer has completed its run cycle, as described herein.

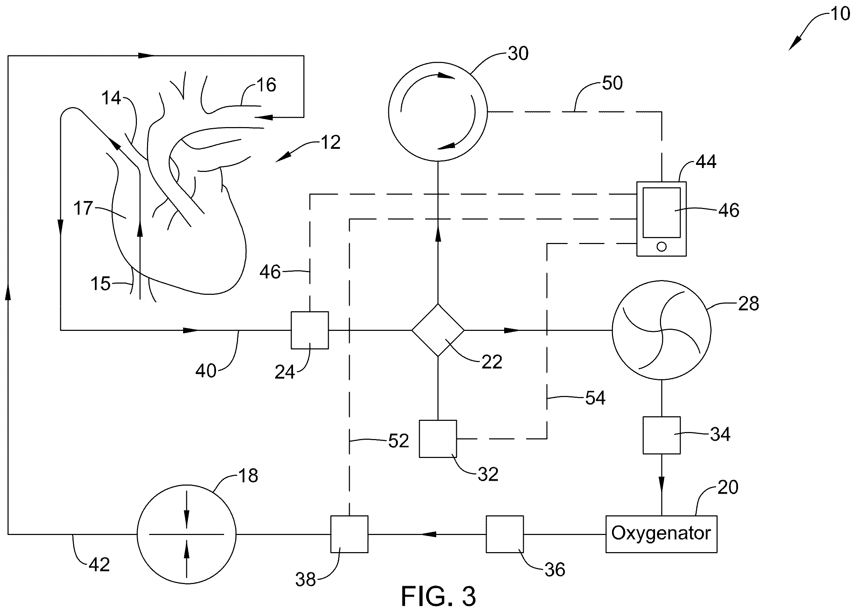

FIG. 3 illustrates another example configuration of the extracorporeal perfusion system 10 described herein. Like FIG. 1, the example configuration of the extracorporeal perfusion system 10 of FIG. 3 includes an oxygenator 20, an arterial clamp 18, an arterial flow sensor 36, an arterial pressure sensor 34, a pump 28 (e.g., arterial pump), a first venous air bubble sensor 24, an arterial air bubble sensor 38, an air trap 22 (e.g., air bubble trap), a roller pump 30 and a control unit 44. Additionally, the extracorporeal perfusion system 10 shown in FIG. 3 may include one or more blood pathways 40, 42 extending between various components of the extracorporeal perfusion system 10.

Additionally, FIG. 3 illustrates that the extracorporeal perfusion system 10 may include a first level sensor 32 coupled to the air trap 22 (e.g., air bubble trap). Further, the first level sensor 32 may be in communication with the control unit 44. For example, FIG. 3 depicts the first level sensor 32 in communication with the control unit 44 via a dashed line 54.

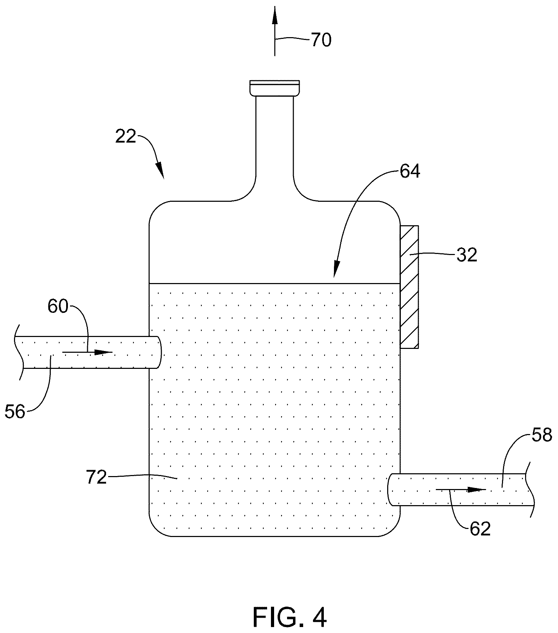

It can be appreciated that the first level sensor 32 may be configured to monitor the level of fluid (e.g., blood) in the air trap 22 (e.g., air bubble trap). For example, FIG. 4 illustrates the first level sensor 32 coupled to the air trap 22 (e.g., air bubble trap). FIG. 4 illustrates that fluid (e.g., blood) may pass into the air trap 22 (e.g., air bubble trap) via a fluid inlet 56 (fluid flowing into the air trap 22 (e.g., air bubble trap) is shown by the directional arrow 60 in FIG. 4) and exit the air trap 22 (e.g., air bubble trap) via a fluid outlet 58 (fluid flowing out of the air trap 22 (e.g., air bubble trap) is shown by the directional arrow 62 in FIG. 4). As discussed herein, the first venous bubble sensor 24 may sense air in the fluid which is passing into the air trap 22 (e.g., air bubble trap) via the fluid inlet 56. Accordingly, when the first venous bubble sensor 24 senses air in the fluid passing into the air trap 22 (e.g., air bubble trap), it may start the roller pump 30 (shown in FIG. 3). The roller pump 30 may generate a vacuum which pulls the air out of the fluid 72 (e.g., blood) flowing through the air trap 22 (e.g., air bubble trap). The direction of the air being pulled out of the air trap 22 (e.g., air bubble trap) is depicted by the directional arrow 70 in FIG. 4.

Further, FIG. 4 illustrates that the level 64 of fluid 72 in the air trap 22 (e.g., air bubble trap) may change as air is removed via the vacuum force generated by the roller pump 30. For example, it can be appreciated that the fluid level 64 of the fluid 72 may rise within the air trap 22 (e.g., air bubble trap) as air is removed from the fluid 72. It can be further appreciated that first level sensor 32 may be configured to sense the level 64 of the fluid 72 and stop the roller pump 30 when the fluid level 64 reaches a given threshold. In other words, as air is removed from the fluid 72 via the action of the roller pump 30, the first level sensor 32 may be programmed to send a signal to the roller pump 30 (via the control unit 44) to cease pumping when the level 64 of the fluid 72 rises above a set threshold. In some examples, the fluid level 64 at which the first level sensor 32 sends the signal to the roller pump 30 to stop pumping may be input by a user. In other examples, the fluid level 64 at which the first level sensor 32 sends the signal to the roller pump 30 to stop pumping may be pre-programmed by a user.

It can be appreciated that with sensing and communication from both the first venous bubble sensor 24 and the first level sensor 32, the roller pump 30 may run for the minimum amount of time to remove the undesired air from the fluid 72 (e.g., blood) in the air trap 22 (e.g., air bubble trap) which may then exit the air trap 22 (e.g., air bubble trap) and enter the arterial blood pathway 42.

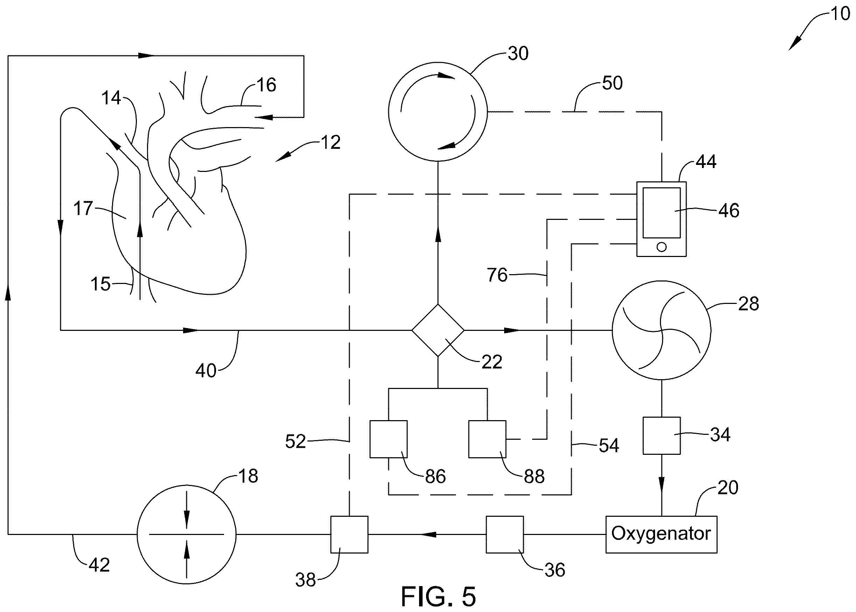

FIG. 5 illustrates another example configuration of the extracorporeal perfusion system 10 described herein. Like FIG. 1 and FIG. 3, the example configuration of the extracorporeal perfusion system 10 of FIG. 5 includes an oxygenator 20, an arterial clamp 18, an arterial flow sensor 36, an arterial pressure sensor 34, a pump 28 (e.g., arterial pump), an arterial air bubble sensor 38, an air trap 22 (e.g., air bubble trap), a roller pump 30 and a control unit 44. Additionally, the extracorporeal perfusion system 10 shown in FIG. 5 may include one or more blood pathways 40, 42 extending between various components of the extracorporeal perfusion system 10.

Additionally, FIG. 5 illustrates that the extracorporeal perfusion system 10 may include a first level sensor 86 and a second level sensor 88 coupled to the air trap 22 (e.g., air bubble trap). The first level sensor 86 and the second level sensor 88 may be in communication with the control unit 44. For example, FIG. 5 depicts the first level sensor 86 in communication with the control unit 44 via a dashed line 54 and the second level sensor 88 in communication with the control unit 44 via a dashed line 76 .

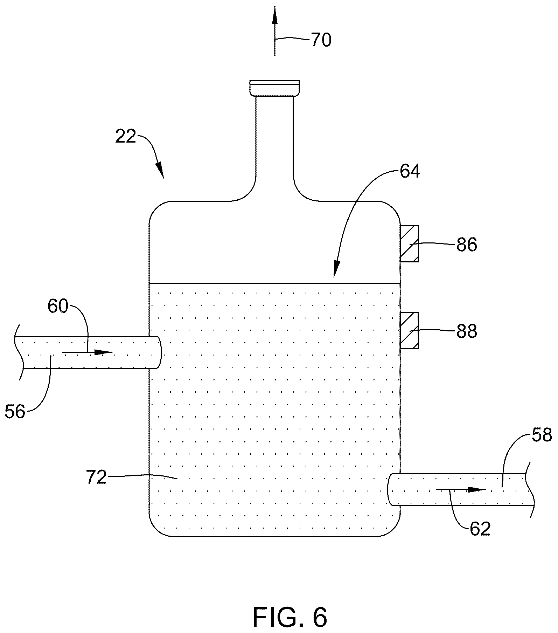

It can be appreciated that the first level sensor 86 may be configured to monitor a maximum level of fluid (e.g., blood) in the air trap 22 (e.g., air bubble trap) while the second level sensor 88 may be configured to monitor a minimum level of fluid (e.g., blood) in the air trap 22 (e.g., air bubble trap). For example, FIG. 6 illustrates the first level sensor 86 and the second level sensor 88 coupled to the air trap 22 (e.g., air bubble trap). FIG. 6 illustrates that fluid (e.g., blood) may pass into the air trap 22 (e.g., air bubble trap) via a fluid inlet 56 (fluid flowing into the air trap 22 (e.g., air bubble trap) is shown by the directional arrow 60 in FIG. 4) and exit the air trap 22 (e.g., air bubble trap) via a fluid outlet 58 (fluid flowing out of the air trap 22 (e.g., air bubble trap) is shown by the directional arrow 62 in FIG. 4). Like that described with respect to FIG. 4, FIG. 6 illustrates that the level 64 of fluid 72 in the air trap 22 (e.g., air bubble trap) may change as air is removed via the vacuum force generated by the roller pump 30. For example, it can be appreciated that the level 64 of the fluid 72 may rise within the air trap 22 (e.g., air bubble trap) as air is removed from the fluid 72. Additionally, it can be appreciated that the level 64 of the fluid 72 may lower within the air trap 22 (e.g., air bubble trap) as the amount of air increases in the fluid 72.

It can be further appreciated that first level sensor 86 may be configured to sense the level 64 of the fluid 72 and start the roller pump 30 when the fluid level 64 falls below a minimum threshold. In other words, as air is introduced into the fluid 72 (e.g., during placement of the arterial cannula and/or venous cannula or via holes in the tubing of the blood pathways 40, 42) the first level sensor 86 may be programmed to send a signal to the roller pump 30 (via the control unit 44) to start pumping when the level 64 of the fluid 72 falls under a minimum threshold. In some examples, the fluid level 64 at which the first level sensor 86 sends the signal to the roller pump 30 to start pumping may be input by a user. In other examples, the fluid level 64 at which the first level sensor 86 sends the signal to the roller pump 30 to start pumping may be pre-programmed by a user.

Further, it can be appreciated that starting the roller pump 30 may generate a vacuum which pulls the air out of the fluid 72 (e.g., blood) flowing through the air trap 22 (e.g., air bubble trap). The direction of the air being pulled out of the air trap 22 (e.g., air bubble trap) is depicted by the directional arrow 70 in FIG. 6. It can be appreciated that the that the fluid level 64 in the air trap 22 (e.g., air bubble trap) may rise as air is removed via the vacuum force generated by the roller pump 30.

It can be further appreciated that second level sensor 88 may be configured to sense the level 64 of the fluid 72 and stop the roller pump 30 when the fluid level 64 reaches a maximum threshold. In other words, as air is removed from the fluid 72 via the action of the roller pump 30, the second level sensor 88 may be programmed to send a signal to the roller pump 30 (via the control unit 44) to cease pumping when the level 64 of the fluid 72 rises above a set, maximum threshold. In some examples, the fluid level 64 at which the second level sensor 88 sends the signal to the roller pump 30 to stop pumping may be input by a user. In other examples, the fluid level 64 at which the second level sensor 88 sends the signal to the roller pump 30 to stop pumping may be pre-programmed by a user.

It can be appreciated that with sensing and communication from both the first level sensor 86, the second level sensor 88 and the roller pump 30 may run for the minimum amount of time to remove the undesired air from the fluid 72 (e.g., blood) in the air trap 22 (e.g., air bubble trap) which may then exit the air trap 22 (e.g., air bubble trap) and enter the arterial blood pathway 42.

It should be understood that this disclosure is, in many respects, only illustrative. Changes may be made in details, particularly in matters of shape, size, and arrangement of steps without exceeding the scope of the disclosure. This may include, to the extent that it is appropriate, the use of any of the features of one example embodiment being used in other embodiments. The disclosure's scope is defined in the language in which the appended claims are expressed.

## Claims

1. An extracorporeal blood treatment system, comprising:
an extracorporeal blood pathway between a venous side of the heart and an arterial side of the heart;
an air trap positioned along the blood pathway;
a first sensor positioned adjacent the bubble trap;
a second sensor positioned adjacent the bubble trap; and
wherein a first signal sent by the first sensor is configured to activate air removal from the air trap;
wherein a second signal sent by the second sensor is configured to deactivate air removal from the air trap.

2. The extracorporeal blood treatment system of claim 1, wherein the first sensor is configured to sense a presence of air in blood flowing along the blood pathway.

3. The extracorporeal blood treatment system of claim 2, wherein the second sensor is configured to sense a change in a gas volume fraction of blood passing along a tubing pathway extending between the air trap and an air removal element.

4. The extracorporeal blood treatment system of claim 3, wherein the air removal element includes a vacuum device.

5. The extracorporeal blood treatment system of claim 3, wherein the air removal element includes a roller pump.

6. The extracorporeal blood treatment system of any one of the preceding claims, wherein the first sensor is positioned upstream of the air trap.

7. The extracorporeal blood treatment system of claim 1, further comprising a tubing pathway having a first end attached to the air trap and a second end attached to an air removal element, and wherein the second sensor is positioned along the tubing pathway.

8. The extracorporeal blood treatment system of claim 1, further comprising a control unit in communication with the first sensor, the second sensor and the air removal element.

9. The extracorporeal blood treatment system of claim 8, wherein the first sensor is configured to transmit a first signal to the control unit, wherein the second sensor is configured to transmit a second signal to the control unit, wherein the air removal element is configured to receive a third signal corresponding to the first signal from the control unit, and wherein the air removal element is configured to receive a fourth signal corresponding to the second signal from the control unit, and wherein the air control element is configured to adjust a vacuum force in the air trap after receiving the third signal, the fourth signal or both the third and the fourth signals.

10. The extracorporeal blood treatment system of claim 1, wherein the second sensor includes a first level sensor, the first level sensor configured to sense a minimum fluid level of blood in the air trap.

11. The extracorporeal blood treatment system of claim 10, wherein the first level sensor is configured to send a signal corresponding to the minimum fluid level of blood in the air trap.

12. The extracorporeal blood treatment system of claim 11, further comprising a third sensor, and wherein the third sensor includes a second level sensor, wherein the second level sensor is configured to sense a maximum fluid level of blood in the air trap.

13. The extracorporeal blood treatment system of claim 12, wherein the second level sensor is configured to send a signal corresponding to the maximum fluid level of blood in the air trap.

14. An extracorporeal blood treatment system, comprising:
an extracorporeal blood pathway between a venous side of the heart and an arterial side of the heart;
an air trap positioned along the blood pathway;
a first sensor positioned adjacent the air trap;
a second sensor positioned adjacent the air trap; and
an air removal element coupled to the air trap;
a control unit in communication with the first sensor, the second sensor and the air removal element;
wherein the air removal element is configured to activate after receiving a first signal from the first sensor;
wherein the air removal element is configured to at least partially deactivate after receiving a second signal from the second sensor.

15. An extracorporeal blood treatment system, comprising:
an extracorporeal blood pathway between a venous side of the heart and an arterial side of the heart;
an air trap positioned along the blood pathway;
a first sensor positioned upstream of the air trap;
a tubing pathway extending between the air trap and a roller pump;
a second sensor positioned along the tubing pathway; and
a control unit in communication with the first sensor, the second sensor and the roller pump;
wherein the first sensor is configured to send a first signal to the control unit, and wherein the first signal corresponds to an amount of air present in blood flowing within the blood pathway;
wherein the second sensor is configured to send a second signal to the control unit, and wherein the second signal corresponds to an amount of air present in the tubing pathway;
wherein the roller pump is configured to activate after receiving a third signal from the control unit, and wherein the third signal corresponds to the first signal received from the first sensor;
wherein the roller pump is configured to at least partially deactivate after receiving a fourth signal from the control unit, and wherein the fourth signal corresponds to the second signal received from the second sensor.
